**Europäisches Patentamt**

**European Patent Office**

**Office européen des brevets**

(19)

(11) Publication number : **0 286 426 B1**

# EUROPEAN PATENT SPECIFICATION

(12)

(45) Date of publication of patent specification : **04.03.92 Bulletin 92/10**

(51) Int. Cl.$^5$ : **A61F 13/04, D06H 7/22**

(21) Application number : **88303159.3**

(22) Date of filing : **08.04.88**

(54) **Non-fraying substrate for use in an orthopaedic casting bandage and a process for its preparation.**

(30) Priority : **10.04.87 US 36928**

(43) Date of publication of application : **12.10.88 Bulletin 88/41**

(45) Publication of the grant of the patent : **04.03.92 Bulletin 92/10**

(84) Designated Contracting States : **BE CH DE ES FR GB IT LI NL**

(56) References cited :
EP-A- 0 144 119
EP-A- 0 181 731
FR-A- 2 444 110

(73) Proprietor : **SMITH AND NEPHEW CASTINGS INC.**
**232 Lindbergh Avenue**
**Livermore California (US)**

(72) Inventor : **Papulski, Keith Alexander**
**6398 Hansen Drive**
**Plaesanton California (US)**
Inventor : **Hicks, John Kenneth**
**92 The Boulevard**
**Hull North Humberside (GB)**

(74) Representative : **Cole, William Gwyn**
**Corporate Patents Department Smith and Nephew Research Limited Gilston Park Harlow Essex CM20 2RQ (GB)**

EP 0 286 426 B1

Jouve, 18, rue Saint-Denis, 75001 PARIS

## Description

The invention relates to a resin-coated, knitted glass fibre substrate suitable for use as an orthopaedic bandage which has a cut end prevented from fraying by the presence of a polymer strip. The present invention also relates to a method of preventing the cut end of a resin-coated, knitted glass fibre substrate from fraying by impregnating into the resin-coated substrate a polymer strip and then cutting the substrate through the polymer strip.

Many orthopaedic bandages which are commercially available are prepared from knitted substrates which have been coated or impregnated with a curable resin. Conventionally the resin-coated substrate is manufactured as a long length, up to 500m long, which is subsequently cut across its width at predetermined intervals to provide bandage length pieces which are packaged as rolls in moisture proof packages. In use the substrate is removed from the package and unrolled to construct the orthopaedic cast. Some commercially available orthopaedic bandages employ as a substrate a knitted glass fibre fabric. One major disadvantage which is apparent during the manufacture of orthopaedic bandages from resin-coated glass fibre substrates is that when the substrate is cut across its width the cut end begins to fray as the yarns are cut and adjacent loops unravel. The presence of a frayed end in the subsequently formed bandage may prevent the formation of a smooth cast and give a poor appearance to the outer turn of the cast. A frayed end may also be sharp and irritating to the skin if present on the inside turn of the cast.

One method of preventing the fraying of a fibreglass knit tape has been described in United States Patent No. 4609578. This method requires the fibreglass knit tape to be subjected to treatment at elevated temperatures prior to coating with a resin. This treatment is said to set the yarns of the fibreglass knit which reduces fraying after any subsequent cutting procedures. However, heat treatment of fibreglass fibres at the temperatures envisaged in that patent removes the lubricant from the surface of the fibres so that the fibres may subsequently be required to be treated with a second coat of lubricant and may cause a decrease in the tensile strength of the tape. If this occurs it may cause reduced strength of any cast formed from the heat treated fabric. The heat treatment is also costly in terms of energy requirement and time of processing.

A resin-coated, knitted glass fibre substrate has now been achieved which is prevented from fraying at a cut end by the presence of a polymer strip at that cut end. The effect may be achieved by applying the polymer strip to the substrate so as to impregnate or coat the glass fibres and then cutting through the polymer strip. The polymer may be applied to the substrate in a way which does not involve the use of high temperature treatment of the glass fibre substrate and in a way which does not affect the elasticity or tensile strength of the substrate. It is also an advantage that the polymer strip may be applied to the resin-coated substrate which avoids the need for a search and detection system to determine the correct position for cutting which would be the case if a polymer strip is applied to the substrate before coating with the resin.

Accordingly the present invention provides a resin-coated, knitted glass fibre substrate which is prevented from fraying at a cut end by the presence of a polymer strip impregnated into the resin coated substrate at said cut end.

The substrate may be any glass fibre fabric which has the properties required for use as an orthopaedic bandage as regards to extensibility and strength. The glass fibre fabrics may be available in two knit-types namely a Raschel knit and a tricot knit. Suitable glass fibre fabrics are described in for example United States Patent Nos. 3686725, 3787272, 3793686 and 4609578.

Suitable polymers for forming the polymer strip include those polymers which will impregnate or coat the glass fibres of the substrate. Preferred polymers are those which can be applied directly to the resin-coated substrate and will disperse through the resin and impregnate the glass fibres. Preferred polymers are thermoplastic polymers which include polyolefins such as polyethylene, polypropylene and polyurethanes. A suitable polymer strip may comprise a single polymer, a mixture of polymers or a mixture of polymer with various compounding aids for example fillers, plasticisers, antioxidants and the like. A suitable polymer strip may be formed from a hot melt adhesive.

Suitably the strip may have a width of from 7 to 20mm more suitably 8 to 15mm and preferably 9 to 12mm for example 10mm.

Suitably the strip may be impregnated at a weight of from 0.05 to 0.25g per 2.5cm of the width of substrate and preferably from 0.1 to 0.2g per 2.5cm of the width of substrate.

Aptly the resin used to coat the glass fibre substrate may be a cold water curable isocyanate terminated polyurethane prepolymer system. Among suitable polyurethane prepolymer systems are those identified in United States Patent Nos. 4411262, 4427002, 4433680 and 4574793. Particularly preferred are those systems disclosed and claimed in United States Patent Nos. 4427002 and 4574793.

Accordingly in another aspect the present invention provides a method of preventing the cut ends of a resin-coated, knitted glass fibre substrate from fraying which method comprises impregnating the resin-coated glass fibre substrate at the end to be cut with a polymeric material to form a strip therein and then cutting the substrate through the strip.

The polymer may be impregnated into the resin-coated glass fibre substrate in one of two ways. Firstly in a less preferred method the polymer may be dissolved in a volatile organic solvent which may be sprayed or painted onto the resin-coated substrate. The substrate is then heated to remove the solvent and deposit the polymer. Suitable solvents include acetone and methylene chloride. Secondly, the preferred method where the polymer is thermoplastic, is to apply the polymer as a hot melt coat. In this method the polymer is extruded in a molten form across the width of the substrate as a strip or a bead using conventional hot melt extrusion apparatus. If the polymer is extruded as a bead, then a heated paddle may be used to spread the polymer into a strip. If the resin-coated glass fibre substrate is carried on a cooled surface, the polymer strip sets rapidly and the cutting operation may follow directly on the extrusion process.

The resin-coated glass fibre substrate which has been treated with polymer may be cut using conventional scissors or preferably by using a sonic knife. The presence of the polymer strip has two further advantages. Firstly the substrate may be cut using a sonic knife which is not possible for untreated glass fibre substrate. The use of a sonic knife provides a smoother cut than conventional scissors. Secondly untreated resin tends to adhere to and foul the blades of conventional scissors. Treated resin significantly reduces the incidence of fouling of the blades.

Example 1

A water curable polyurethane resin system comprising a polyurethane prepolymer described in United States No. 4574793 as prepolymer A and containing methane sulphonic acid as stabiliser and bis(2,6 dimethylmorpholino)diethyl ether as catalyst was coated onto an untreated knitted glass fibre fabric substrate using the process described in United States Patent No. 4427002. The resin-coated glass fibre fabric substrate is wound onto a master roll which is approximately 25cm in width and is 500m in length.

A predetermined length of the resin-coated substrate is then wound onto a core and the thermoplastic polymer is applied as a 10mm strip across the width of the bandage. The polymer is applied as a hot melt coating by extrusion through a heated die as a thin bead across the width of the resin coated substrate. This bead is then impregnated into the substrate as a strip, 10mm wide, by means of a heated paddle located behind the heated die. The die may be moved by and operated by manual control. The polyurethane coated substrate is supported on a refrigerated plate which causes the thermoplastic compound to set quickly. The substrate is then cut along approximately the centre of strip using a sonic knife so as to separate the bandage length from the rest of the master roll. The winding of the bandage length is completed and the bandage is packaged in a moisture-proof pouch.

A further predetermined length of the resin-coated substrate may be then unrolled from the master roll and wound onto a core, the cutting process repeated as described above. By repeating this process, bandage-size lengths of substrate may be taken from the master roll, each end of the bandage being prevented from fraying by the presence of a strip of the thermoplastic polymer.

Example 2

A process was carried out as described in Example 1 except that the thermoplastic polymer was a hot melt adhesive.

## Claims

1. A resin-coated knitted glass fibre substrate suitable for use as an orthopaedic casting bandage which is prevented from fraying at a cut end by the presence of a polymer strip impregnated into the resin coated substrate at said cut end.

2. A resin-coated, knitted glass fibre substrate according to claim 1 in which the strip is from 3 to 20mm wide and is present at a weight per unit length of substrate of from 0.02 to 0.1g per cm of the substrate.

3. A resin-coated, knitted glass fibre substrate according to claims 1 or 2 in which the strip is formed from a thermoplastic polymer.

4. A resin-coated knitted glass fibre substrate according to claims 1 to 3 in which the strip is formed from a hot melt adhesive.

5. A resin-coated, knitted glass fibre substrate according to claim 1 in which the resin is an isocyanate-terminated polyurethane prepolymer.

6. A resin-coated, knitted glass fibre substrate according to claim 1 in which the polymer strip has been applied at the end to be cut by impregnating the resin-coated substrate with polymer to form a strip therein and then cutting through the polymer strip.

7. A resin-coated, knitted glass fibre substrate according to claim 6 the polymer strip is applied as a hot melt coating.

8. A resin-coated, knitted glass fibre substrate according to claim 6 in which the polymer strip is applied as a solution of the polymer in a volatile organic solvent and then removing the solvent.

9. A resin-coated, knitted glass fibre substrate according to claim 6 in which the substrate is cut by means of a sonic knife.

10. A method of preventing the cut ends of a resin-coated, knitted glass fibre substrate from fraying which method comprises impregnating the resin-coated glass fibre substrate at the end to be cut with a polymeric material to form a strip therein and then cutting the substrate through the strip.

11. A method according to claim 10 in which the strip is formed from a thermoplastic polymer.

12. A method according to claim 11 in which the strip is applied as a hot melt coating.

13. A method according to claim 12 in which the strip is from 8 to 15mm wide and is impregnated at a weight of from 0.05 to 0.25g per 2.5cm of the width of substrate.

14. A method according to claim 11 in which the cut is made by an ultrasonic knife.

15. A method according to claim 11 in which the strip is applied by dissolving the thermoplastic polymer in a volatile organic solvent, painting or spraying the solution onto the resin-coated substrate and removing the solvent to form the strip.

16. A method according to claim 10 in which the resin is a cold water curable isocyanate terminated polyurethane prepolymer.

## Revendications

1. Substrat de fibres de verre tricoté revêtu de résine utilisable en tant que bandage orthopédique dont l'effilochage d'une extrémité coupée est empêché par la présence d'une bande de polymère imprégné dans le substrat revêtu de résine au niveau de cette extrémité coupée.

2. Substrat de fibres de verre tricoté revêtu de résine suivant la revendication 1, dans lequel la bande a une largeur de 3 à 20 mm et est présent à raison d'un poids par unité de longueur du substrat de 0,02 à 0,1 g/cm de substrat.

3. Substrat de fibres de verre tricoté revêtu de résine suivant la revendication 1 ou la revendication 2, dans lequel la bande est formée à partir d'un polymère thermoplastique.

4. Substrat de fibres de verre tricoté revêtu de résine suivant les revendications 1 à 3, dans lequel la bande est formée à partir d'un adhésif de type hot melt.

5. Substrat de fibres de verre tricoté revêtu de résine suivant la revendication 1, dans lequel la résine est un prépolymère de polyuréthane à terminaison isocyanate.

6. Substrat de fibres de verre tricoté revêtu de résine suivant la revendication 1, dans lequel la bande de polymère a été appliquée sur l'extrémité devant être coupée par imprégnation du substrat revêtu de résine avec du polymère pour former une bande dans celui-ci, puis découpe à travers la bande de polymère.

7. Substrat de fibres de verre tricoté revêtu de résine suivant la revendication 6, dans lequel la bande de polymère est appliquée en tant que revêtement hot melt.

8. Substrat de fibres de verre tricoté revêtu de résine suivant la revendication 6, dans lequel la bande de polymère est appliquée sous forme d'une solution du polymère dans un solvant organique volatil, le solvant étant ensuite éliminé.

9. Substrat de fibres de verre tricoté revêtu de résine suivant la revendication 6, dans lequel le substrat est découpé au moyen d'un couteau sonique.

10. Procédé pour empêcher l'effilochage des extrémités coupées d'un substrat de fibres de verre tricoté revêtu de résine, lequel comprend l'imprégnation du substrat de fibres de verre revêtu de résine à l'extrémité devant être coupée avec un matériau polymère de façon à former une bande dans celui-ci puis la coupe du substrat à travers la bande.

11. Procédé suivant la revendication 10, dans lequel la bande est formée à partir d'un polymère thermoplastique.

12. Procédé suivant la revendication 11, dans lequel la bande est appliquée sous forme de revêtement hot melt.

13. Procédé suivant la revendication 12, dans lequel la bande a une largeur de 8 à 15 mm et est imprégnée à un poids de 0,05 à 0,25 g/2,5 cm de largeur de substrat.

14. Procédé suivant la revendication 11, dans lequel la coupe est faite par un couteau ultra sonique.

15. Procédé suivant la revendication 11, dans lequel la bande est appliquée par dissolution du polymère thermoplastique dans un solvant organique volatil, peinture ou pulvérisation de la solution sur le substrat revêtu de résine et élimination du solvant pour former la bande.

16. Procédé suivant la revendication 10, dans lequel la résine est un pré-polymère de polyuréthane à terminaison isocyanate durcissable par l'eau froide.

## Patentansprüche

1. Mit Harz beschichtetes, gewirktes Glasfaser-Trägermaterial, welches für die Verwendung als orthopädische Schienungsbandage geeignet ist, deren Ausfransen an einem Schnittende durch das Vorhandensein eines Polymerstreifens verhindert wird, welcher in das mit Harz beschichtete Trägermaterial an dem geschnittenen Ende imprägniert ist.

2. Mit Harz beschichtetes, gewirktes Glasfaser-Trägermaterial nach Anspruch 1, bei welchem der Streifen eine Breite von 3 - 20 mm aufweist und mit einem Gewicht pro Längeneinheit des Substrats von 0,02 - 0,1 g/cm des Substrats vorhanden ist.

3. Mit Harz beschichtetes, gewirktes Glasfaser-Trägermaterial nach Anspruch 1 oder 2, bei welchem der Streifen aus einem thermoplastischen Polymer gebildet ist.

4. Mit Harz beschichtetes, gewirktes Glasfaser-Trägermaterial nach den Ansprüchen 1 - 3, bei welchem der Streifen aus einem Heißkleber gebildet ist.

5. Mit Harz beschichtetes, gewirktes Glasfaser-Trägermaterial nach Anspruch 1, bei welchem das Harz ein mit endständigem Isozyanat versehenes Polyurethan-Präpolymer ist.

6. Mit Harz beschichtetes, gewirktes Glasfaser-Trägermaterial nach Anspruch 1, bei welchem der Polymerstreifen an dem zu schneidenden Ende durch Imprägnieren des mit Harz beschichteten Trägermaterials mit Polymer angebracht worden ist, um darin einen Streifen zu bilden und dann durch den Polymerstreifen hindurchzuschneiden.

7. Mit Harz beschichtetes, gewirktes Glaserfaser-Trägermaterial nach Anspruch 6, bei welchem der Polymerstreifen als heißschmelzender Überzug aufgebracht ist.

8. Mit Harz beschichtetes, gewirktes Glasfaser-Trägermaterial nach Anspruch 6, bei welchem der Polymerstreifen als eine Lösung des Polymers in einem flüchtigen organischen Lösungsmittel aufgebracht wird und danach das Lösungsmittel entfernt wird.

9. Mit Harz beschichtetes, gewirktes Glasfaser-Trägermaterial nach Anspruch 6, bei welchem das Trägermaterial mittels eines sonischen Messers geschnitten wird.

10. Verfahren zum Verhindern des Ausfransens der geschnittenen Enden einese mit Harz beschichteten, gewirkten Glasfaser-Trägermaterials, wobei das mit Harz beschichtete Glasfaser-Trägermaterial an dem zu schneidenden Ende mit einem polymeres Material imprägniert wird, um in dem Trägermaterial einen Streifen zu bilden und dieses dann durch den Streifen zu schneiden.

11. Verfahren nach Anspruch 10, bei welchem der Streifen aus einem thermoplastischen Polymer gebildet wird.

12. Verfahren nach Anspruch 11, bei welchem der Streifen als heißschmelzender Überzug aufgebracht wird.

13. Verfahren nach Anspruch 12, bei welchem der Streifen 8 - 15 mm breit ist und mit einem Gewicht von 0,05 - 0,25 g pro 2,5 cm der Breite des Trägermaterials imprägniert wird.

14. Verfahren nach Anspruch 11, bei welchem der Schnitt durch ein Ultraschall-Messer ausgeführt wird.

15. Verfahren nach Anspruch 11, bei welchem der Streifen durch Auflösen des thermoplastischen Polymers in einem flüchtigen organischen Lösungsmittel aufgebracht wird und die Lösung auf das mit Harz beschichtete Trägermaterial gestrichen oder gesprüht und das Lösungsmittel entfernt wird, um den Streifen zu bilden.

16. Verfahren nach Anspruch 10, bei welchem das Harz ein in kaltem Wasser härtbares, mit endständigem Isozyanat versehenes Polyurethan-Präpolymer ist.